# EUROPÄISCHE PATENTSCHRIFT

(11) **EP 2 776 493 B1**
(45) Veröffentlichungstag und Bekanntmachung des Hinweises auf die Patenterteilung: **21.03.2018**
(21) Anmeldenummer: 12797742.9
(22) Anmeldetag: 07.11.2012
(51) Int. Cl.: C08J 3/075, A61L 27/52

(54) **HERSTELLUNG VON HYDROGELEN MITTELS DIELS-ALDER REAKTION**
PRODUCTION OF HYDROGELS BY MEANS OF DIELS-ALDER REACTION
PRODUCTION D'HYDROGELS PAR LA RÉACTION DE DIELS-ALDER

(30) Priorität: 08.11.2011 DE 102011117839
(43) Veröffentlichungstag der Anmeldung: 17.09.2014
(73) Patentinhaber: Universität Regensburg, 93053 Regensburg (DE)
(72) Erfinder: GÖPFERICH, Achim, 93161 Sinzing (DE); BRANDL, Ferdinand, 76135 Karlsruhe (DE); KIRCHHOF, Susanne, 88212 Ravensburg (DE)
(74) Vertreter: Vossius & Partner Patentanwälte Rechtsanwälte mbB
(86) Internationale Anmeldenummer: PCT/EP2012/072027
(87) Internationale Veröffentlichungsnummer: WO 2013/068397

(56) Entgegenhaltungen:
- WO-A1-2007/003054
- WO-A1-2012/057751
- US-A1- 2011 077 737
- WEI H L ET AL: "Thermosensitive hydrogels synthesized by fast Diels-Alder reaction in water", POLYMER, ELSEVIER SCIENCE PUBLISHERS B.V, GB, Bd. 50, Nr. 13, 19. Juni 2009 (2009-06-19) , Seiten 2836-2840, XP026159444, ISSN: 0032-3861, DOI: 10.1016/J.POLYMER.2009.04.032 [gefunden am 2009-05-03]
- BRANDL F ET AL: "Poly(ethylene glycol) based hydrogels for intraocular applications", ADVANCED ENGINEERING MATERIALS, WILEY VCH VERLAG, WEINHEIM, DE, Bd. 9, Nr. 12, 1. Dezember 2007 (2007-12-01), Seiten 1141-1149, XP002579229, ISSN: 1438-1656, DOI: 10.1002/ADEM.200700221 [gefunden am 2007-12-19] in der Anmeldung erwähnt

## Beschreibung

Gegenstand der vorliegenden Erfindung sind Hydrogele sowie deren Herstellung durch Quervernetzung von Makromonomeren mittels Diels-Alder Reaktion. Die beschriebenen Hydrogele, die in-situ gelieren können, eignen sich u.a. als Biomaterial für medizinische Anwendungen, als Gerüstmaterial für lebende Zellen und als Trägersystem für die kontrollierte Freigabe von Arzneistoffen. Für die Herstellung der Hydrogele werden Makromoleküle mit Dienen und Dienophilen funktionalisiert; die Anzahl der funktionellen Gruppen (Dien bzw. Dienophil) beträgt mindestens zwei pro Makromonomer. Bei der anschließend ablaufenden [4+2]-Cycloaddition reagieren die beschriebenen Makromonomere in wässriger Lösung außer- oder innerhalb des tierischen oder menschlichen Organismus zu kovalent quervernetzten Hydrogelen. Durch Modifikation der Makromonomere mit kurzen Peptiden (z.B. enzymatisch spaltbare Sequenzen und/oder die Zelladhäsion fördernde Peptide) können die Hydrogele an die Erfordernisse der jeweiligen Anwendung angepasst werden. Die zur Quervernetzung verwendete Diels-Alder Reaktion ermöglicht darüber hinaus die Funktionalisierung der Hydrogele mit weiteren molekularen Komponenten, die der Erkennung, Markierung oder aktiven Interaktion mit ihrer Umgebung dienen.

Arzneistoffe biogenen Ursprungs, wie z.B. Peptide, Proteine oder Nukleinsäuren, gewinnen in zahlreichen innovativen Therapieansätzen zunehmend an Bedeutung. Um ihr volles therapeutisches Potential entfalten zu können, müssen die meisten Peptide, Proteine oder Nukleinsäuren in geeigneten Trägersystemen "verpackt" werden. Hydrogele bieten ideale Voraussetzungen, um den strengen Anforderungen an Trägersysteme für Arzneistoffe biogenen Ursprungs gerecht zu werden. Bei Hydrogelen handelt es sich um dreidimensionale Netzwerke, die durch Quervernetzung von natürlichen oder synthetischen Polymeren erhalten werden. Sie sind in der Lage, ein Vielfaches ihres Trockengewichts an Wasser aufzunehmen und zu binden. Aufgrund ihrer ausgezeichneten Biokompatibilität und ihrer hohen Permeabilität für Nährstoffe und Stoffwechselprodukte, werden Hydrogele bereits in zahlreichen biomedizinischen Anwendungen eingesetzt. Besonders interessant für biomedizinische Anwendungen sind in situ-gelierende Hydrogele, die als flüssige Polymerlösung injiziert werden können und sich dann am Applikationsort zu einem Gel verfestigen. Die Applikation mittels minimalinvasiver Methoden wird dabei als besonders schonend für den Patienten angesehen. Ziel dieser Strategie ist es, die enthaltenen Peptide, Proteine oder Nukleinsäuren nach erfolgter Gelierung aus dem Hydrogel freizusetzen und lokal oder systemisch zur Wirkung zu bringen.

Zur Herstellung von Hydrogelen sind zahlreiche, auf Polymeren biologischen (z.B. Polysaccharide) oder synthetischen Ursprungs (z.B. Polyacrylate oder Polyethylenglykol) basierende Ansätze in der Literatur beschrieben. Die Quervernetzung der einzelnen Polymerketten kann sowohl durch chemische (kovalente) als auch physikalische Bindungen (z.B. hydrophobe oder ionische Wechselwirkungen) erfolgen. Kovalent quervernetzte Hydrogele sind hinsichtlich ihrer hohen Stabilität und weitgehenden Unempfindlichkeit gegenüber äußeren Einflüssen (z.B. Änderungen des pH-Werts oder der lonenstärke) besonders vorteilhaft für biomedizinische Anwendungen. Die in der Polymerchemie gebräuchlichen Quervernetzungsreaktionen (wie z.B. radikalische Polymerisationen, Polykondensationen oder Polyadditionen) sind jedoch häufig auf toxische Kupplungsreagenzien, Katalysatoren oder Photoinitiatoren angewiesen. Die hergestellten Hydrogele müssen folglich aufwendigen und damit teuren Reinigungsschritten unterzogen werden, um sie von toxischen Verunreinigungen und/oder nicht polymerisierten Monomeren zu befreien. Die eigentlich erstrebenswerte Injektion als Polymerlösung und anschließende Quervernetzung am Applikationsort verbietet sich meist von selbst. Eine unkontrollierte und damit aktivitätsmindernde Reaktion mit anwesenden Peptiden, Proteinen oder Nukleinsäuren kann aufgrund der Vielzahl an vorhandenen funktionellen Gruppen (z.B. Alkohole, Amine, Carbonsäuren usw.) oftmals nicht vermieden werden. Diese beiden Nachteile stehen einer Anwendung von chemisch quervernetzten Hydrogelen als Trägersysteme für Arzneistoffe biogenen Ursprungs häufig entgegen.

Sogenannte Click-Reaktionen stellen eine interessante Alternative zu konventionellen Quervernetzungsreaktionen dar. Click-Reaktionen sind chemische Reaktionen, die unter einfachen Reaktionsbedingungen (z.B. im wässrigen Milieu) und mit höchster Effizienz ausschließlich zu den gewünschten Reaktionsprodukten führen. Eine Reaktion mit anderen funktionellen Gruppen (z.B. von biogenen Arzneistoffen) kann aufgrund der Orthogonalität der an den Click-Reaktionen beteiligten Gruppen weitestgehend vermieden werden. Unter dem Konzept der Orthogonalität versteht man allgemein die freie Kombinierbarkeit funktioneller Gruppen, ohne dass es zu einer unerwünschten Reaktion zwischen den beteiligten Gruppen kommt. Die bekannte und in der organischen Chemie häufig durchgeführte Kupfer(I) katalysierte Azid/Alkin-Cycloaddition (1,3-dipolare Cycloaddition, Huisgen-Reaktion) besitzt jedoch ähnliche Nachteile wie konventionelle Quervernetzungsreaktionen: aufgrund der hohen Toxizität des eingesetzten Kupfer(I) Katalysators ist es unerlässlich, die hergestellten Hydrogele aufwendigen und damit teuren Reinigungsschritten zu unterziehen. Eine direkte Injektion in den tierischen oder menschlichen Organismus scheidet auch hier aus.

Die Diels-Alder Reaktion erfüllt alle Kriterien einer Click-Reaktion (u.a. Durchführbarkeit unter einfachen Bedingungen und hohe Effizienz) und stellt eine äußerst interessante Möglichkeit zur kovalenten Quervernetzung von Makromonomeren dar. Bei der Diels-Alder Reaktion handelt es sich um eine einstufige [4+2]-Cycloaddition zwischen einem Dien und einem Dienophil. Typischerweise kommen Dienophile zum Einsatz, die eine C-C-Doppelbindung aufweisen. Es können jedoch auch Verbindungen als Dienophile in einer Diels-Alder Reaktion umgesetzt werden, die eine Doppelbindung zwischen einem Kohlenstoffatom und einem Heteroatom (z.B. N, O oder S) oder zwischen zwei Heteroatomen (z.B. N-N oder N-O) aufweisen, wie z.B. ein Aldehyd, ein Keton, ein Imin oder ein Thioketon. Die Diels-Alder Reaktion kann schematisch beispielsweise durch die folgende Reaktionsgleichung (1) wiedergegeben werden.

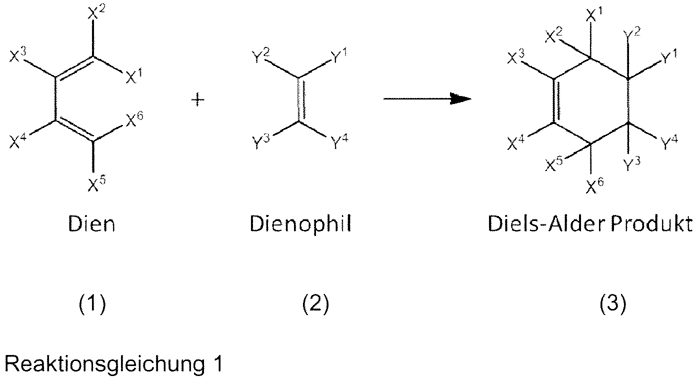

Die Gleichung zeigt eine allgemeine schematische Darstellung der Diels-Alder [4+2]-Cycloaddition zwischen einem Dien der Formel (1) und einem Dienophil der Formel (2) unter Bildung eines Diels-Alder Produkts der Formel (3). Die Reste X¹ bis X⁶ und Y¹ bis Y⁴ stellen beliebige Atome oder Molekülreste dar.

Die Reaktion läuft im wässrigen Milieu beschleunigt ab; auf den Zusatz von Katalysatoren oder Initiatoren kann vollständig verzichtet werden. Durch geeignete Wahl der Substituenten X¹ bis X⁶ und Y¹ bis Y⁴ kann die Reaktionsgeschwindigkeit beeinflusst werden. Zur Beschleunigung der Reaktion werden für X¹ bis X⁶ elektronenschiebende Substituenten (Gruppen mit +I bzw. +M-Effekt, z.B. Alkylgruppen) gewählt; für Y¹ bis Y⁴ werden bevorzugt elektronenziehende Substituenten (Gruppen mit -I bzw. -M-Effekt) eingesetzt. Beispielsweise können Diene der Formel (1) Wasserstoffatome, Kohlenwasserstoffreste, (wie z.B. Alkylreste), oder Alkoxyreste (insbesondere Methoxyreste) unabhängig voneinander als Reste X¹ bis X⁶ tragen. Insbesondere die Reste X¹ und X⁶ können darüber hinaus auch unter Bildung eines carbocyclischen oder heterocyclischen fünf- oder sechsgliedrigen Ringsystems, wie einem Furanring, einem Cyclopentadienring oder einem 1,3-Cyclohexadienring, miteinander verknüpft sein.

Dienophile der Formel (2) können z.B. an zwei oder drei der Positionen Y¹ bis Y⁴ ein Wasserstoffatom oder einen Kohlenwasserstoffrest, z.B. einen Alkylrest, tragen, und an der/den verbleibenden Position(en) einen elektronenziehenden Rest, wie eine Aldehydfunktion -C(O)H, eine Ketofunktion -C(O)R, eine Esterfunktion -C(O)OR, eine Cyanogruppe oder eine Nitrogruppe, wobei R ein Kohlenwasserstoffrest, z.B. ein Alkylrest, ist. Gut geeignet als Dienophile sind auch Verbindungen der Formel (2) wobei Y² und Y³ ein Wasserstoffatom oder einen Kohlenwasserstoffrest, z.B. einen Alkylrest, darstellen, und Y¹ und Y⁴ unter Bildung eines Maleinsäureanhydrids, eines Maleimids oder eines 1,4-Chinons miteinander verbunden sind.

Wie für Additionsreaktionen üblich, so führt auch die Diels-Alder Reaktion nicht zur Freisetzung von niedermolekularen und potenziell toxischen Nebenprodukten. Darüber hinaus können unerwünschte Nebenreaktionen mit anderen funktionellen Gruppen weitestgehend ausgeschlossen werden. Der Grund hierfür ist die Orthogonalität der für die Quervernetzungsreaktion eingesetzten funktionellen Gruppen (Dien und Dienophil). Diese funktionellen Gruppen reagieren unter den gewählten Bedingungen ausschließlich untereinander und können mit anderen funktionellen Gruppen (z.B. Alkohole, Amine, Carbonsäuren usw.) beliebig kombiniert werden; auf den Einsatz von Schutzgruppen kann verzichtet werden. Dies ist für die angestrebte Anwendung der beschriebenen Hydrogele von besonderem Wert, da unkontrollierte und damit aktivitätsmindernde Reaktionen mit anwesenden Peptiden, Proteinen oder Nukleinsäuren weitestgehend ausgeschlossen sind. Neben den bereits genannten Vorteilen sind die verwendeten Makromonomere (Dien und Dienophil) auch äußerst lagerstabil; dies grenzt sie von anderen häufig eingesetzten Derivaten (z.B. aktivierte Carbonsäuren oder Thiole) ab.

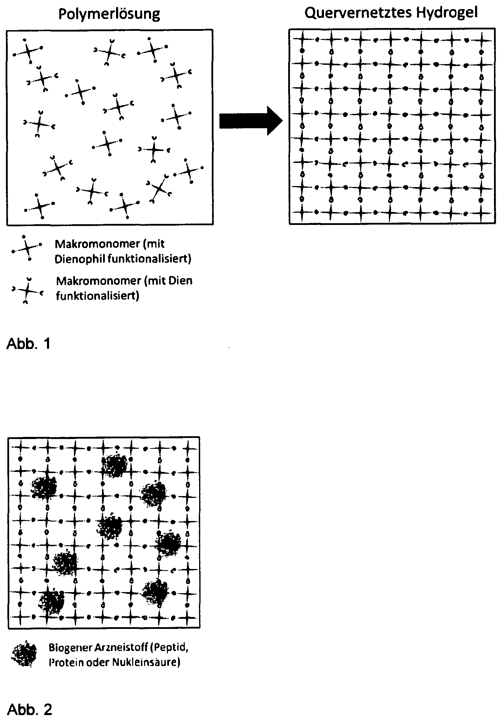

Nicht zuletzt aufgrund der dargelegten Vorteile wurde die Diels-Alder Reaktion bereits in der Polymerchemie eingesetzt. So finden sich in der Literatur Beispiele, in denen die Diels-Alder Reaktion zur Herstellung von Elastomeren eingesetzt wurde. Darunter versteht man elastisch verformbare Kunststoffe, deren Glasübergangstemperatur unterhalb ihrer Einsatztemperatur liegt. Wesentlicher Unterschied zu den hier beschriebenen Hydrogelen ist ihr äußerst geringer Wassergehalt. Ihre möglichen Einsatzgebiete unterscheiden sich deshalb stark von den in dieser Erfindung vorgeschlagenen Anwendungsgebieten. In der Literatur finden sich auch Anwendungsbeispiele, in denen die Diels-Alder Reaktion zur Herstellung von Hydrogelen verwendet wurde. So wurden z.B. hochmolekulare Polyacrylamide, Poylacrylate und Polyvinylpyrrolidone mittels Diels-Alder Reaktion vernetzt. Bei dieser Art der Quervernetzung entstehen typischerweise Hydrogele mit hoher Solfraktion und wenig definierter Architektur; Maschenweite, Quellungsgrad und mechanische Eigenschaften sind vergleichsweise schlecht kontrollierbar. Zusätzliche Nachteile der existierenden Hydrogele sind die Verwendung von nicht zugelassenen Polymeren sowie die nicht vorhandene Bioabbaubarkeit. Jeder dieser Punkte verhindert üblicherweise die Anwendung der Hydrogele in biomedizinischen Bereichen. Weiterhin sind die in der Literatur beschriebenen Hydrogele oftmals optisch nicht transparent. Dies steht z.B. einem Einsatz in ophthalmologischen Anwendungen entgegen. In einem anderen Ansatz wurde Hyaluronsäure mittels Diels-Alder Reaktion quervernetzt. Der entscheidende Nachteil ist hierbei die Verwendung von Polymeren biologischen Ursprungs. Dies lässt eine hohe Variabilität des Ausgangsmaterials, schlecht reproduzierbare Herstellungsvorgänge, hohe Produktionskosten sowie ein allergenes Potenzial der hergestellten Hydrogele erwarten. Weiterhin kann die eingesetzte Hyaluronsäure nur schwer chemisch verändert werden. Die Anpassung der Hydrogele an die Bedürfnisse der jeweiligen Anwendung (z.B. Abbaubarkeit, Zelladhäsion, Freisetzungsgeschwindigkeit usw.) gestaltet sich hier äußerst schwierig. Gegenstand der Erfindung sind daher ein Verfahren zur Herstellung eines Hydrogels durch kovalente Quervernetzung von Makromonomeren mittels Diels-Alder Reaktion, wobei es sich bei den Makromonomeren um Stern- oder Kammpolymere mit einem Verzweigungsgrad von zwei bis acht handelt, sowie durch dieses Verfahren erhältliche Hydrogele. Vorteilhafterweise sind die hier vorgestellten Komponenten zur Herstellung eines Hydrogels in der Lage, in situ, z.B. nach Applikation in einen Organismus, das Hydrogel zu bilden. Eine schematische Darstellung der Vernetzungsreaktion zeigt Abb. 1.

Makromonomere, die erfindungsgemäß zur Herstellung der Hydrogele eingesetzt werden können, sind Polymere (auch als Makromoleküle bezeichnet), die funktionelle Gruppen aufweisen, aufgrund derer sie als Monomere in einer Polymerisationsreaktion reagieren können. Im Rahmen der vorliegenden Erfindung handelt es sich bei dieser Reaktion dem entsprechend um die Diels-Alder-Reaktion zwischen entsprechend funktionalisierten Makromonomeren, die Dien- bzw. Dienophilfunktionen aufweisen.

Als Ausgangspolymere zur Bereitstellung der Makromonomere werden bevorzugt hydrophile Polymere eingesetzt, insbesondere hydrophile Polymere synthetischen Ursprungs. Besonders bevorzugt sind von der FDA (U.S. Food and Drug Administration) für biomedizinische Anwendungen zugelassene hydrophile Polymere synthetischen Ursprungs. Als Beispiel für ein besonders geeignetes Ausgangspolymer sei Polyethylenglykol (PEG) genannt (auch als Polyethylenoxid (PEO) bezeichnet), wobei die Bezugnahme auf Polyethylenglykol (PEG) bzw. Polyethylenoxid verzweigte Strukturen einschließt. Dieses wasserlösliche, biokompatible, nicht toxische und nicht immunogene Polymer findet bereits breite Anwendung in Biotechnologie und Medizin. Weitere Beispiele von Makromonomeren, die für den Einsatz im Rahmen der Erfindung geeignet sind, umfassen Polyvinylalkohol (PVA), Polypropylenoxid (PPO), Copolymere aus Ethylenoxid und Propylenoxid (PEO-co-PPO), Poly(hydroxyethylmethacrylat) (pHEMA), Hyaluronsäure, Dextran, Kollagen, Chitosan, Alginat, Cellulose und Cellulosederivate, wie Carboxymethylcellulose. Die verwendeten Makromonomere zeichnen sich vorzugsweise durch ein relativ geringes Molekulargewicht (typischerweise 2 - 20 kDa) aus. Darüber hinaus ist eine definierte Architektur vorteilhaft. Es kommen Stern- oder Kammpolymere zum Einsatz, beispielsweise solche mit einem Verzweigungsgrad von zwei bis acht, bevorzugt drei bis acht und insbesondere vier bis acht. Der Verzweigungsgrad bezeichnet dabei die Anzahl der Polymerketten, die vom Verzweigungspunkt bzw. den Verzweigungspunkten des Polymers ausgehen. Demgemäß sind hydrophile Stern- oder Kammpolymere synthetischen Ursprungs besonders bevorzugt, welche den vorstehend angegebenen Verzweigungsgrad aufweisen. Die folgenden Strukturformeln zeigen beispielhaft verzweigte PEG-Moleküle, die für den Einsatz im Rahmen der vorliegenden Erfindung gut geeignet sind. Die Zahl der Wiederholungseinheiten n kann so eingestellt werden, dass sich das gewünschte Molekulargewicht des Gesamtmoleküls ergibt.

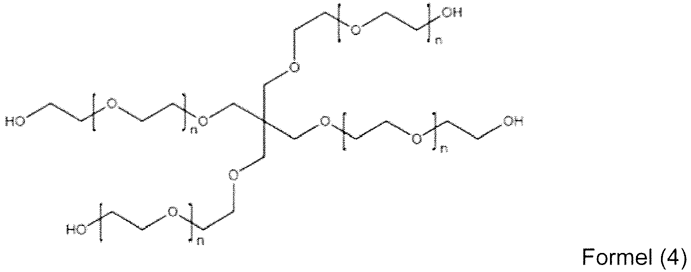

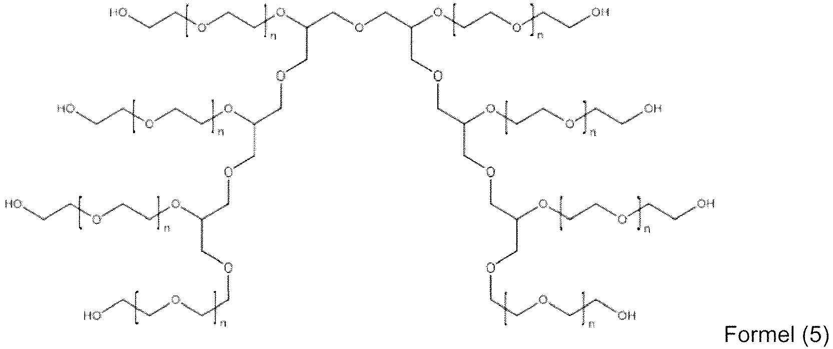

Formel (4) zeigt ein 4-armig verzweigtes PEG-Molekül (Verzweigungsgrad 4), hier auch als "4armPEG-OH" bezeichnet. Es kann in unterschiedlichen Molekulargewichten, beispielsweise mit einem Molekulargewicht von 10.000 Da (4armPEG10k-OH) eingesetzt werden.

Formel (5) zeigt ein 8-armig verzweigtes PEG-Molekül (Verzweigungsgrad 8), hier auch als "8armPEG-OH" bezeichnet. Es kann ebenfalls in unterschiedlichen Molekulargewichten, beispielsweise mit einem Molekulargewicht von 10.000 Da (8armPEG10k-OH) eingesetzt werden.

Für die Bereitstellung der Makromonomere als Reaktanden für eine Diels-Alder Quervernetzungsreaktion werden die gewählten Ausgangspolymere mit mindestens zwei Dienen bzw. mindestens zwei Dienophilen funktionalisiert. Ein Makromonomer sollte also bevorzugt entweder nur Dienfunktionen oder nur Dienophilfunktionen aufweisen, da dies eine kontrollierte Reaktion zwischen Dien- und Dienophilkomponenten erleichtert. Sollen Makromonomere mit gemischten Dien- und Dienophilfunktionen eingesetzt werden, so wäre dafür Sorge zu tragen, dass diese nicht bereits während ihrer Bereitstellung oder Lagerung unkontrolliert intra- oder intermolekulare Reaktionen eingehen. Während beide Ausgangspolymere mit jeweils mindestens zwei Dienfunktionen bzw. mindestens zwei Dienophilfunktionen funktionalisiert werden, sind typischerweise die beiden Reaktanden so aufeinander abgestimmt, dass die Quervernetzung effektiv ablaufen kann. Weist beispielsweise ein Makromonomer nur zwei funktionelle Gruppen für die Diels-Alder Reaktion auf, so wird es in der Regel mit einem anderen Makromonomer kombiniert, das drei oder mehr funktionelle Gruppen besitzt. Bevorzugt wird jedes Ausgangspolymermolekül mit mindestens drei, insbesondere mit mindestens vier Dienen bzw. mit mindestens drei, insbesondere mit mindestens vier Dienophilen funktionalisiert, um so ein Makromonomer zu bilden. Es ist insbesondere bevorzugt, dass jeder Arm der eingesetzten Stern- oder Kammpolymere endständig mit einem Dien bzw. Dienophil funktionalisiert ist.

Zur Einführung einer Dien- bzw. Dienophilfunktion können bekannte Verfahren zur kovalenten Anbindung, z.B. unter Bildung einer Ester oder Amidbindung eingesetzt werden. Bezugnehmend auf die schematische Darstellung in Reaktionsgleichung 1 können beispielsweise ein oder mehrere Substituenten X¹ bis X⁶ bzw. Y¹ bis Y⁴ zur Anknüpfung eines Diens bzw. Dienophils an ein Polymer dienen.

Wie vorstehend in der Reaktionsgleichung 1 veranschaulicht, werden als Diene für eine Diels-Alder-Reaktion typischerweise offenkettige oder cyclische Verbindungen eingesetzt, die zwei konjugierte C-C-Doppelbindungen aufweisen, wobei die Doppelbindungen in offenkettigen Verbindungen eine cisoide Konfiguration einnehmen. Beispiele solcher Diene sind solche der vorstehenden Formel (1). Bevorzugt sind Diene, die einen Furanring, einen Cyclopentadienring oder einen 1,3-Cyclohexadienring aufweisen. Dienophile sind typischerweise Verbindungen mit einer C-C-Doppelbindung, die beispielhaft in der vorstehenden Formel (2) beschrieben werden. Es können jedoch auch Verbindungen als Dienophile in einer Diels-Alder Reaktion umgesetzt werden, die eine Doppelbindung zwischen einem Kohlenstoffatom und einem Heteroatom (z.B. N, O oder S) oder zwischen zwei Heteroatomen (z.B. N-N oder N-O) aufweisen, wie z.B. ein Aldehyd, ein Keton, ein Imin oder ein Thioketon. Auch heterozyklische Verbindungen, wie 4-Phenyl-1,2,4-triazolin-3,5-dion (PTAD) können als Dienophil zum Einsatz kommen. Bevorzugt sind Dienophile, die eine Maleinsäureanhydrid-Gruppe, eine Maleimid-Gruppe oder eine 1,4-Chinon-Gruppe umfassen. Insbesondere können elektronenreiche Furyl-Reste als Diene dienen; elektronenarme Maleimid-Gruppen können als Dienophil eingesetzt werden. Durch diese Art der Endgruppenmodifikation mit relativ kleinen Resten ändert sich wenig an der Löslichkeit der derivatisierten Polymere, so dass es sich bei den Makromonomeren, in denen wasserlösliche Polymere eingesetzt werden, nach wie vor um wasserlösliche Verbindungen handeln kann. Die so synthetisierten Makromonomere können außerdem als biokompatibel angesehen werden.

Zur Herstellung der Hydrogele können die synthetisierten Makromonomere separat in einer definierten Menge Wasser oder Pufferlösung (z.B. phosphatgepufferte Kochsalzlösung pH 7,4) gelöst werden. Anschließend können beide Polymerlösungen miteinander gemischt und bei definierter Temperatur (z.B. 37 °C) inkubiert werden, wobei sich durch die ablaufende [4+2]-Cycloaddition ein kovalent quervernetztes Hydrogel ausbildet. Mit diesem unkomplizierten Verfahren lässt sich eine Vielzahl von Hydogelen herstellen, die exakt an die Bedürfnisse der jeweiligen Anwendung angepasst werden können.

Die Quervernetzung der Makromonomere erfolgt durch schrittweise Polymerisation (sog. "Step Growth Polymerization") ausschließlich unter Verwendung der Diels-Alder [4+2]-Cycloaddition; radikalische Polymerisationsreaktionen und/oder physikalische Quervernetzungsprinzipien (z.B. elektrostatische oder hydrophobe Wechselwirkungen) kommen nicht zum Einsatz. Beispielhaft ist die Reaktion der Makromere in der folgenden Reaktionsgleichung 2 gezeigt, nach der mit einem Furyl-Rest als Dien funktionalisierte PEG-Makromonomere und mit einem Maleimid-Rest als Dienophil funktionalisierte PEG-Makromonomere in einer [4+2]-Cycloaddition zu kovalent quervernetzten Hydrogelen reagieren. Die unterbrochene Bindung, die an dem Ende der Moleküle gezeigt ist, das dem Dien bzw. Dienophil entgegengesetzt ist, verdeutlicht, dass lediglich ein Ausschnitt der Makromonomere gezeigt ist, da für die Vernetzung wie vorstehend beschrieben noch weitere reaktive Gruppen vorhanden sind (vgl. auch **Abb. 1**).

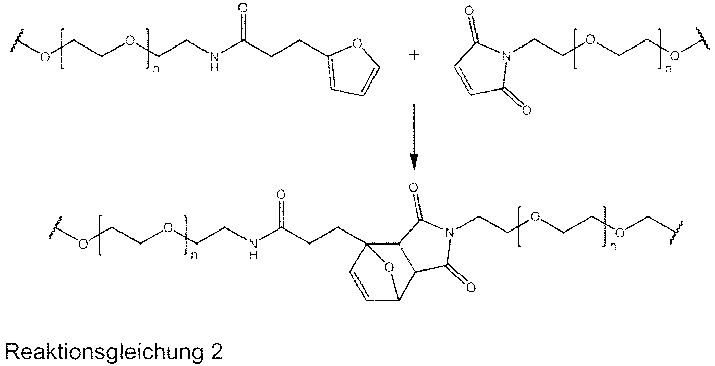

Die durch die Diels-Alder Reaktion erhältlichen Hydrogele weisen eine definierte Architektur, kontrollierbare Maschenweite und eine äußerst geringe Solfraktion auf. Der Polymergehalt der Hydrogele liegt typischerweise unter 25 % (w/v, bezogen auf g/ml); der Wassergehalt kann im gequollenen Zustand bis zu 95 % (w/v) betragen. Weiterhin zeichnen sich die Hydrogele bevorzugt durch exzellente optische Transparenz aus, was eine Vielzahl von biomedizinischen (z.B. ophthalmologischen) und technischen Anwendungen ermöglicht. Das beschriebene Verfahren bzw. das daraus resultierende Hydrogel eignet sich insbesondere für die Bereitstellung eines Gerüstmaterials für lebende Zellen, z.B. implantierte oder eingewanderte Zellen, oder für die Breitstellung eines Trägersystems zur kontrollierten Freigabe von Arzneistoffen, wie Arzneistoffe biogenen Ursprungs, bevorzugt von Peptiden, Proteinen oder Nukleinsäuren in biologisch aktiver Form (vgl. **Abb. 2**). Bevorzugte Ausführungsformen der Erfindung betreffen daher (a) ein Hydrogel wie vorstehend beschrieben, das erhältlich ist durch Quervernetzung von Makromonomeren mittels Diels-Alder [4+2]-Cycloaddition und das zusätzlich lebende Zellen enthält, und (b) ein Hydrogel wie vorstehend beschrieben, das erhältlich ist durch Quervernetzung von Makromonomeren mittels Diels-Alder [4+2]-Cycloaddition, das einen Arzneistoff enthält. Der Arzneistoff kann ausschließlich durch das gebildete Netzwerk im Hydrogel fixiert sein. Er kann aber auch im Hydrogel in kovalent gebundener Form vorliegen. In Bezug auf die Ausführungsform (b) lassen sich zwei bevorzugte Szenarien b1) und b2) unterscheiden, die mit herkömmlichen Quervernetzungsreaktionen nur schwer realisierbar sind:
b1) Die funktionellen Gruppen von anwesenden Peptiden, Proteinen oder Nukleinsäuren (z.B. Alkohole, Amine, Carbonsäuren usw.) sind gegenüber der Diels-Alder Quervernetzungsreaktion weitestgehend inert; eine unkontrollierte und somit aktivitätsmindernde Vernetzung von Peptiden, Proteinen oder Nukleinsäuren wird effektiv verhindert. Arzneistoffe biogenen Ursprungs lassen sich bereits während der Quervernetzung in Hydrogele "verpacken", was eine äußerst hohe Beladungseffizienz gewährleistet. Gleichzeitig ist jedoch ihre rasche Freisetzung in biologisch aktiver Form gewährleistet.
b2) Peptide, Proteine oder Nukleinsäuren können durch geeignete Linkermoleküle so derivatisiert werden, dass sie während der Quervernetzungsreaktion reversibel an das Gelnetzwerk angebunden werden. Geeignete Linkermoleküle umfassen dabei beispielsweise eine Dien oder Dienophilfunktionalität. Durch die kovalente Anbindung an das Gelnetzwerk werden anwesende Peptide, Proteine oder Nukleinsäuren im Hydrogel festgehalten. Entsprechend der Abbaukinetik der Linkermoleküle können sie beispielsweise verzögert an die Umgebung abgegeben werden. Dieser Ansatz ist besonders vielversprechend, wenn eine Therapie mit biogenen Arzneistoffen über einen längeren Zeitraum hinweg angestrebt wird.

Die erfindungsgemäßen Hydrogele besitzen typischerweise gute Biokompatibilität. In der Regel können sie im Organismus abgebaut werden, nachdem sie ihren Zweck erfüllt haben. Falls gewünscht, kann der Abbau zusätzlich durch Modifikation der Makromonomere beschleunigt werden. Sind die verwendeten Ausgangspolymere nicht von vornherein bioabbaubar (wie z.B. PEG), so können diese beispielsweise durch den Einbau einer hydrolytisch spaltbaren Sequenz oder einer enzymatisch spaltbaren Sequenz modifiziert werden. So kann z.B. gewährleistet werden, dass die erfindungsgemäßen Hydrogele nach Applikation in einen Organismus dort wieder abgebaut werden können. Diese Bioabbaubarkeit kann bei der Verwendung als Gerüstmaterial für lebende Zellen oder als Trägersubstanz für Arzneimittel von Vorteil sein. Natürlich ist der Einbau hydrolytisch oder enzymatisch spaltbarer Sequenzen auch dann geeignet, die Abbaugeschwindigkeit eines erfindungsgemäßen Hydrogels zusätzlich zu steuern bzw. zu beschleunigen, wenn ein Ausgangspolymer verwendet wird, das als solches im Organismus eines Patienten abgebaut werden kann.

Als Beispiel für eine hydrolytisch spaltbare Sequenz können z.B. Oligomere der Milchsäure genannt werden. Auch einzelne Estergruppen können als hydrolytisch spaltbare Gruppen die Abbaukinetik des Hydrogels steuern. Als Beispiele für eine enzymatisch spaltbare Sequenz seien bekannte Peptidsequenzen genannt, die durch Matrix-Metalloproteasen (MMP) gespalten werden können (z.B. das Pro-Leu-Gly-Leu-Trp-Ala-Arg-Motiv). Der Einbau einer hydrolytisch oder enzymatisch spaltbaren Sequenz kann vorteilhafterweise zwischen der Endgruppe des Ausgangspolymers und dem anzuknüpfenden Dien bzw. Dienophil erfolgen. Beispielhaft wird diese Vorgehensweise mit der Struktur der folgenden Formel (6) veranschaulicht, in der zwischen ein PEG als Polymer und eine funktionelle Gruppe mit einem Furylrest als Dien eine spaltbare Sequenz Z eingefügt wurde.

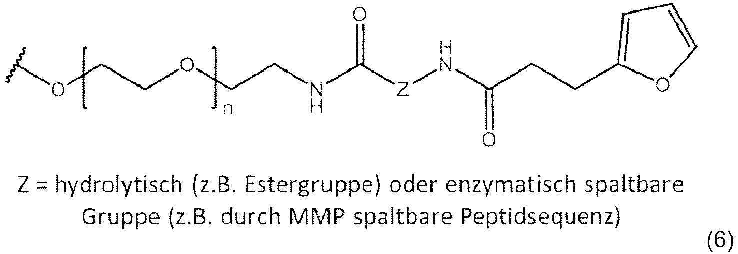

Das Einführen einer spaltbaren Gruppe gelingt problemlos, da zur Funktionalisierung der Ausgangspolymere Reaktionen verwendet werden können, die z.B. mit den Standardmethoden der Peptidsynthese kompatibel sind. Nach Quervernetzung der Makromonomere mittels Diels-Alder Reaktion entstehen hydrolytisch bzw. enzymatisch abbaubare Hydrogele. Die Verwendung von enzymatisch spaltbaren Sequenzen hat den Vorteil, dass der Abbau der quervernetzten Hydrogele nicht mit konstanter Geschwindigkeit abläuft sondern von den biologischen Gegebenheiten am Applikationsort (z.B. Enzymexpression) abhängt. Durch Variation von Anzahl und Art der enzymatisch spaltbaren Sequenzen kann die Abbaurate der Hydrogele je nach Anwendung entweder beschleunigt oder verzögert werden. Diese Möglichkeit stellt eine zusätzliche Innovation gegenüber bereits bekannten Hydrogelen dar, deren Abbaurate nicht oder nur äußerst schwer kontrolliert werden kann.

Neben spaltbaren Sequenzen können auch andere Moleküle oder Stoffe, wie z.B. Peptidsequenzen zur Förderung der Zelladhäsion (z.B. das Arg-Gly-Asp-Ser Motiv), Cyclodextrine, Farbstoffe zur Detektion oder Nanopartikel, direkt an das Gelgerüst angebunden werden. Dazu müssen diese Moleküle entweder mit einem Dien oder einem Dienophil funktionalisiert werden. Diese hohe Flexibilität in der Ausgestaltung der Hydrogele eröffnet zahlreiche Anwendungsmöglichkeiten im Bereich des Tissue Engineering. Durch den Einbau von speziellen der Zelladhäsion dienenden Peptidsequenzen, wie z.B. des Arg-Gly-Asp-Ser oder des Tyr-Ile-Gly-Ser-Arg-Motivs, lassen sich Adhäsion, Proliferation, Migration und Differenzierung von implantierten oder eingewanderten Zellen gezielt beeinflussen. Darüber hinaus können aufgrund der definierten Architektur der verwendeten Makromonomere und der hohen Effizienz der Diels-Alder Quervernetzungsreaktion Maschenweite, Quellungsgrad und mechanische Eigenschaften der Hydrogele kontrolliert werden. Dies ist ebenfalls im Tissue Engineering von besonderer Bedeutung, da auch die physikochemischen Eigenschaften des Zellträgers einen Einfluss auf Adhäsion, Proliferation, Migration und Differenzierung von implantierten oder eingewanderten Zellen haben.

Generell können die erfindungsgemäßen Hydrogele weitere Hilfsstoffe enthalten. Dabei kann es sich auch um molekulare Komponenten zur Erkennung, Markierung oder aktiven Interaktion mit der Umgebung handeln.

Wie vorstehend dargelegt besitzen die beschriebenen Hydrogele zahlreiche Anwendungsmöglichkeiten. Beispiele für geeignete Anwendungen umfassen (a) ein Trägersystem für einen Arzneistoff, insbesondere eines biogenen Arzneistoff, wie ein Peptid, ein Protein oder eine Nukleinsäure; (b) ein Gerüstmaterial für lebende Zellen, insbesondere in das Gerüstmaterial implantierte oder eingewanderte Zellen; (c) Hydrogele für ophthalmologische Anwendungen, d.h. insbesondere zur Behandlung von Erkrankungen am Auge; (d) ein Füllmaterial, insbesondere für biomedizinische Anwendungen, bei denen typischerweise das Hydrogel als solches einem Patienten appliziert wird. Diese Anwendungsmöglichkeiten sind insbesondere von Interesse im Rahmen der Behandlung, insbesondere der therapeutischen Behandlung, von Mensch oder Tier.

Aufgrund des eingesetzten Reaktionsmechanismus zur Bildung der Hydrogele sind die erfindungsgemäßen Gele in der Lage, in situ, d.h. am Applikationsort in einem lebenden Organismus eines zu behandelnden Patienten (Mensch oder Tier) zu gelieren. Der Einsatz von Hilfsstoffen, z.B. Katalysatoren, oder von Aktivierungsenergie ist dabei in der Regel nicht notwendig. Die Makromonomere können daher bequem in Form einer Polymerlösung appliziert, z.B. injiziert werden, um sich dann am Applikationsort zu einem Gel zu verfestigen. Beispiele für Polymerlösungen sind eine Lösung, die ein Gemisch der Makromonomere enthält, oder zwei getrennte Lösungen, die die Dienmakromonomere bzw. die Dienophilmakromonomere enthalten. Bevorzugte Polymergehalte von Polymerlösungen, die für die in-situ Gelierung besonders geeignet sind, liegen im Bereich von 1 bis 30 %, insbesondere 2,5 bis 20 % (w/v, bezogen auf g/ml)..

Zur Applikation des Hydrogels können beide Komponenten für die Bildung des Gels, d.h. die Dienmakromonomere und die Dienophilmakromonomere, also unmittelbar vor dem Einbringen in einen menschlichen oder tierischen Organismus, beispielsweise vor einer Injektion, gemischt werden. Die Mischung kann vorteilhafterweise in Form einer wässrigen Lösung bereitgestellt werden. Weitere gewünschte Bestandteile, wie ein Arzneistoff und/oder konventionelle pharmazeutische Hilfsstoffe, können der Mischung ebenfalls beigefügt werden. Alternativ können die Komponenten, ggf. zusammen mit einem Arzneistoff und/oder einem oder mehreren Hilfsstoffen, mit Hilfe einer Zwei-Kammer-Spritze injiziert werden. Auch hier ist es vorteilhaft, die zu injizierenden Lösungen als wässrige Lösungen bereit zu stellen. Schließlich besteht natürlich auch die Möglichkeit, die Komponenten zeitlich aufeinander folgend zu applizieren, z.B. durch kurz aufeinander folgende Injektionen an den gleichen Applikationsort.

Die Quervernetzung der Makromonomere erfolgt dann in situ, also direkt am Applikationsort im lebenden Organismus. Auf diese Weise ist eine hohe Variabilität der äußeren Form der Hydrogele gewährleistet; die äußere Form der Hydrogele passt sich den anatomischen Gegebenheiten am Applikationsort an. Die erfindungsgemäßen Hydrogele bilden häufig dreidimensionale Strukturen mit einem Volumen im Mikro- bis Milliliterbereich, z.B. 1 µl bis 10 ml. Sie sind jedoch auch zur Beschichtung von Oberflächen geeignet, wobei aufgrund der gewünschten Quervernetzung hier typischerweise Beschichtungsdicken von mindestens 500 nm bis 1 mm, insbesondere 1 µm bis 1mm bevorzugt sind Die Applikation mittels minimalinvasiver Methoden ist besonders schonend für den Patienten, da keine größeren Hautschnitte erforderlich sind. Weiterhin ist es dadurch möglich, die Hydrogele auch in anatomisch schwer zugänglichen Geweben oder Körperhöhlen anzuwenden. Als Beispiele seien exemplarisch das Augeninnere, das Innenohr, die Stirnhöhle, die Nebenhöhlen oder die Zahnpulpa genannt. Die Sterilität der Hydrogele kann durch Sterilfiltration der polymeren Ausgangslösungen gewährleistet werden.

Die erfindungsgemäßen Hydrogele sind demnach auch geeignet für ein Verfahren zur kontrollierten Verabreichung eines Arzneistoffs an einen zu behandelnden Patienten, umfassend die Applikation eines wie vorstehend beschriebenen Hydrogels oder der zur Bildung des Hydrogels notwendigen Makromonomere zusammen mit dem Arzneistoff an einen Patienten.

Die erfindungsgemäßen Hydrogele sind auch geeignet für ein Verfahren zum Aufbau von Gewebe im Rahmen des Tissue Engineering, umfassend die Applikation eines wie vorstehend beschriebenen Hydrogels oder der zur Bildung des Hydrogels notwendigen Makromonomere zusammen mit den für den Gewebeaufbau notwendigen lebenden Zellen an einen Patienten.

Bevorzugte Ausführungsformen der Erfindung sind in den folgenden Punkten zusätzlich zusammengefasst:
1. Hydrogel, das durch kovalente Quervernetzung von Makromonomeren mittels Diels-Alder [4+2]-Cycloaddition hergestellt wird.
2. Hydrogel nach Punkt 1, wobei für die Herstellung mindestens ein Makromonomer mit mindestens zwei Dienfunktionen und mindestens ein Makromonomer mit mindestens zwei Dienophilfunktionen eingesetzt werden.
3. Hydrogel nach Punkt 1 oder 2, das aus hydrophilen Stern- oder Kammpolymeren synthetischen Ursprungs gebildet ist, insbesondere aus Stern- oder Kammpolymeren mit einem Molekulargewicht von 2 - 20 kDa.
4. Hydrogel nach einem der vorhergehenden Punkte, das aus Polyethylenglykol (PEG) besteht.
5. Hydrogel nach einem der vorherigen Punkte, das nach Verabreichung der flüssigen Vorstufen unmittelbar am Applikationsort quervernetzt wird.
6. Hydrogel nach einem der vorherigen Punkte, das durch Einbau hydrolytisch oder enzymatisch spaltbarer Sequenzen bioabbaubar ist.
7. Hydrogel nach einem der vorherigen Punkte, das weitere molekulare Komponenten zur Erkennung, Markierung oder aktiven Interaktion mit der Umgebung enthält.
8. Hydrogel nach einem der vorherigen Punkte, das biogene Arzneistoffe (z.B. Peptide, Proteine oder Nukleinsäuren) enthält.
9. Hydrogel nach Punkt 8, das biogene Arzneistoffe (z.B. Peptide, Proteine oder Nukleinsäuren) in kovalent gebundener Form enthält.
10. Hydrogel nach einem der vorherigen Punkte, das lebende Zellen enthält.
11. Hydrogel nach einem der vorherigen Punkte, das als Füllmaterial für biomedizinische Anwendungen eingesetzt werden kann.
12. Hydrogel nach einem der vorherigen Punkte, das als Trägersystem insbesondere für die kontrollierte Freigabe von Arzneistoffen eingesetzt werden kann.
13. Hydrogel nach einem der vorherigen Punkte, das als Gerüstmaterial für implantierte oder eingewanderte Zellen eingesetzt werden kann.
14. Hydrogel nach einem der vorhergehenden Punkte geeignet zur Verwendung in ophthalmologischen Anwendungen.

### Beispiele

### Herstellungsbeispiel 1: Funktionalisierung von sternförmigem PEG mit Furyl-Resten

Die Beschreibung der Synthese erfolgt exemplarisch für sternförmiges PEG (Verzweigungsgrad vier, Molekulargewicht 10 kDa, 4armPEG10k-OH). Zur Herstellung des Ausgangsmaterials wurde 4armPEG10k-OH zunächst wie in der Literatur beschrieben (z.B. Brandl, F., Henke, M., Rothschenk, S., Gschwind, R., Breunig, M., Blunk, T., Tessmar, J. and Göpferich, A. (2007), Poly(Ethylene Glycol) Based Hydrogels for Intraocular Applications. Adv. Eng. Mater., 9: 1141-1149) in das entsprechende Amin umgewandelt (4armPEG10k-NH₂). Anschließend wurden 224 mg (1,6 mmol) 3-(2-Furyl)propionsäure, 184 mg (1,6 mmol) *N*-Hydroxysuccinimid und 330 mg (1,6 mmol) *N*,*N*'-Dicyclohexylcarbodiimid in 10 ml 1,4-Dioxan gelöst (Lösung A). Das Reaktionsgemisch wurde für 6 h bei Raumtemperatur gerührt. In einem separaten Gefäß wurden 2 g (0,2 mmol) 4armPEG10k-NH₂ und 134 mg (1,6 mmol) Natriumhydrogencarbonat in 10 ml Wasser gelöst (Lösung B). Lösung A wurde filtriert, mit Lösung B vereinigt und bei 50 °C über Nacht gerührt. Am nächsten Tag wurde das Lösungsmittel entfernt und der Rückstand in 20 ml Wasser aufgenommen. Das Rohprodukt wurde viermal mit je 20 ml Dichlormethan extrahiert. Die vereinigten organischen Phasen wurden über wasserfreiem Natriumsulfat getrocknet und am Rotationsverdampfer auf ca. 5 ml eingeengt. Das Produkt wurde bei 0 °C durch tropfenweise Zugabe von 50 ml Diethylether ausgefällt. Der entstandene Niederschlag wurde mittels Filtration gesammelt, mit kaltem Diethylether gewaschen und unter Vakuum getrocknet (80 % Ausbeute).
¹H-NMR (CDCl₃, 300 MHz): δ 2.50 ppm (t, 8H,-NHC(O)C*H*₂CH₂-), 2.97 ppm (t, 8H,-NHC(O)CH₂*CH*₂-), 3.39 ppm (s, 8H, RC*CH*₂O-), 3.62 ppm (s, *-*O*CH₂CH₂*-), 6.00 ppm (s, 4H, Ar), 6.25 ppm (s, 4H, Ar), 7.27 ppm (s, 4H, Ar)

### Herstellungsbeispiel 2: Funktionalisierung von sternförmigem PEG mit ETPI

Die Beschreibung der Synthese erfolgt exemplarisch für sternförmiges PEG (Verzweigungsgrad vier, Molekulargewicht 10 kDa, 4armPEG10k-OH). Im ersten Schritt wurden 2 g (0,2 mmol) 4armPEG10k-OH in 25 ml Toluol gelöst und mittels azeotroper Destillation getrocknet. Anschließend wurde das restliche Lösungsmittel am Rotationsverdampfer entfernt. Der Rückstand wurde zusammen mit 393 mg (2,4 mmol) 3,6-Epoxy-1,2,3,6-tetrahydrophthalimid (ETPI) und 314 mg (1,2 mmol) Triphenylphosphin in 15 ml Dichlormethan gelöst. Die Mischung wurde im Eisbad auf 0 °C abgekühlt; anschließend wurden 240 µl Diisopropylazodicarboxylat (gelöst in 2,5 ml Dichlormethan) zugetropft. Der Reaktionsansatz wurde bei Raumtemperatur für 48 h gerührt; anschließend wurde das Lösungsmittel am Rotationsverdampfer entfernt. Zur Aufreinigung wurde das Rohprodukt in 50 ml Wasser gelöst und zweimal mit je 50 ml Diethylether gewaschen. Die wässrige Phase wurde vollständig einrotiert. Das Rohprodukt wurde in 5 ml Dichlormethan gelöst und bei 0 °C durch tropfenweise Zugabe von 50 ml Diethylether ausgefällt. Der entstandene Niederschlag wurde mittels Filtration gesammelt, mit kaltem Diethylether gewaschen und unter Vakuum getrocknet (95 % Ausbeute).
¹H-NMR (CDCl₃, 300 MHz): δ 2.84 ppm (s, 4H, exo) 3.39 ppm (s, 8H, RC*CH*₂O-), 3.62 ppm (s, -O*CH₂CH₂-*), 5.24 ppm (s, 4H, endo), 5.29 ppm (s, 4H, exo), 6.40 ppm (s, 4H, -*CH*=*CH*-, endo), 6.50 ppm (s, 4H, *-CH*=*CH*-, exo)

### Herstellungsbeispiel 3: Funktionalisierung von sternförmigen PEG mit Maleimid-Gruppen

Die Beschreibung der Synthese erfolgt exemplarisch für sternförmiges PEG (Verzweigungsgrad vier, Molekulargewicht 10 kDa, 4armPEG10k-OH). 4armPEG10k-OH wurde zunächst wie in Herstellungsbeispiel 2 beschrieben mit ETPI funktionalisiert. Anschließend wurden 2 g des Reaktionsprodukts aus Herstellungsbeispiel 2 in 25 ml Toluol gelöst. Diese Lösung wurde für 2 h unter Argon-Schutzgas bei 120 °C refluxiert. Anschließend wurde das Lösungsmittel am Rotationsverdampfer entfernt. Das Rohprodukt wurde in 5 ml Dichlormethan gelöst und bei 0 °C durch tropfenweise Zugabe von 50 ml Diethylether ausgefällt. Der entstandene Niederschlag wurde mittels Filtration gesammelt, mit kaltem Diethylether gewaschen und unter Vakuum getrocknet (85 % Ausbeute).
¹H-NMR (CDCl₃, 300 MHz): δ 3.39 ppm (s, 8H, RC*CH*₂O-), 3.62 ppm (s, *-OCH₂CH₂-*), 6.69 ppm (s, 8H, -C(O)*CH*=*CH*C(O)-)

### Beispiel 1: Herstellung von Hydrogelen mittels Diels-Alder Reaktion

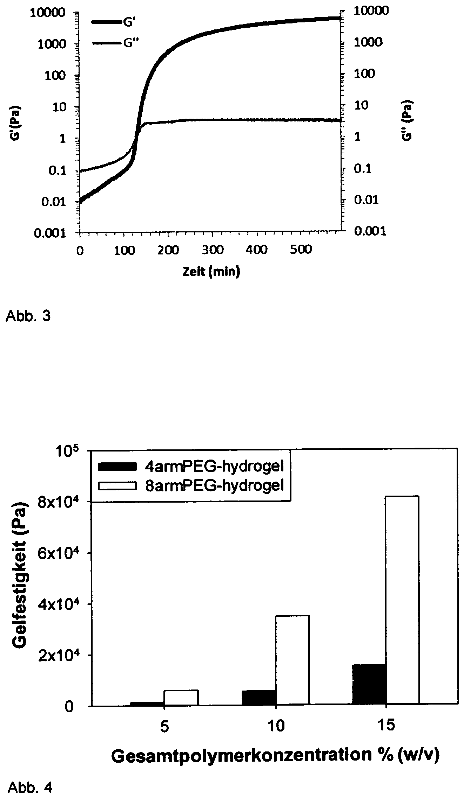

Die Beschreibung der Gelherstellung erfolgt exemplarisch für Makromonomere aus sternförmigem PEG (Verzweigungsgrad vier, Molekulargewicht 10 kDa, 4armPEG10k-OH); der Gesamtpolymergehalt beträgt 10 % (w/v, hier und im Folgenden bezogen auf g/ml, soweit nicht gesondert definiert). Zur Herstellung der Hydrogele wurden mit Furyl-Resten funktionalisierte Makromonomere aus Herstellungsbeispiel 1 und mit Maleimid-Gruppen funktionalisierte Makromonomere aus Herstellungsbeispiel 3 verwendet. Jeweils 50 mg der beiden Makromonomere wurden in je 500 µl Wasser gelöst. Die beiden Polymerlösungen wurden anschließend vereinigt und bei 37 °C inkubiert. Durch die ablaufende [4+2]-Cycloaddition wurden die Makromonomere zu elastischen Hydrogelen quervernetzt. **Abb. 3** zeigt das Rheogramm eines des Hydrogels mit 10 % (w/v) Gesamtpolymergehalt. Die Messung erfolgte am oszillierenden Rheometer (AR 2000 von TA Instruments, 40 mm Platte als Messgeometrie) bei 37 °C.

### Herstellungsbeispiel 4: Funktionalisierung von sternförmigen PEG mit Maleimid-Gruppen

Die Funktionalisierung von sternförmigem PEG mit Verzweigungsgrad vier (Molekulargewicht 10 kDa, 4armPEG10k-OH) wurde in Herstellungsbeispiel 3 beschrieben. Zur Funktionalisierung von sternförmigem PEG mit Verzweigungsgrad acht (Molekulargewicht 10 kDa, 8armPEG10k-OH) wurde ein anderes Verfahren angewendet. Das Ausgangsmaterial (8armPEG10k-OH) wurde zunächst wie in der Literatur beschrieben (z.B. Brandl, F., Henke, M., Rothschenk, S., Gschwind, R., Breunig, M., Blunk, T., Tessmar, J. and Göpferich, A. (2007), Poly(Ethylene Glycol) Based Hydrogels for Intraocular Applications. Adv. Eng. Mater., 9: 1141-1149) in das entsprechende Amin umgewandelt (8armPEG10k-NH₂). Anschließend wurden 2,46 mg 8armPEG10k-NH₂ (0,25 mmol) in 100 ml gesättigter Natriumbicarbonat-Lösung gelöst. Die Lösung wurde im Eisbad auf 0 °C gekühlt und mit 620 mg (4 mmol) *N*-Methoxycarbonylmaleimid versetzt. Nach 30 min Rühren bei 0 °C wurde das Eisbad entfernt und die Lösung langsam auf Raumtemperatur erwärmt. Das Rohprodukt wurde dreimal mit je 115 ml Dichlormethan extrahiert. Die vereinigten organischen Phasen wurden über wasserfreiem Natriumsulfat getrocknet und am Rotationsverdampfer auf ca. 6 ml eingeengt. Das Produkt wurde bei 0 °C durch tropfenweise Zugabe von 60 ml Diethylether ausgefällt. Der entstandene Niederschlag wurde mittels Filtration gesammelt, mit kaltem Diethylether gewaschen und unter Vakuum getrocknet (73 % Ausbeute).
¹H-NMR (CDCl₃, 300 MHz): δ 3.39 ppm (s, 16H, RC*CH*₂O-), 3.62 ppm (s, *-*O*CH₂CH₂-*), 6.69 ppm (s, 16H, -C(O)*CH*=*CH*C(O)-)

### Rheologische Charakterisierung von Hydrogelen

Die Herstellung der Hydrogele erfolgte mit dem in Beispiel 1 beschriebenen Verfahren. Das 4armPEG-Hydrogel wurde aus mit Furyl-Resten funktionalisierten Makromonomeren aus Herstellungsbeispiel 1 und mit Maleimid-Gruppen funktionalisierten Makromonomeren aus Herstellungsbeispiel 3 hergestellt. Das 8armPEG-Hydrogel wurde aus mit Furyl-Resten funktionalisierten Makromonomeren, die entsprechend der Vorgehensweise in Herstellungsbeispiel 1, lediglich unter Verwendung von 8armPEG10k-OH, hergestellt wurden und mit Maleimid-Gruppen funktionalisierten Makromonomeren aus Herstellungsbeispiel 4 hergestellt.

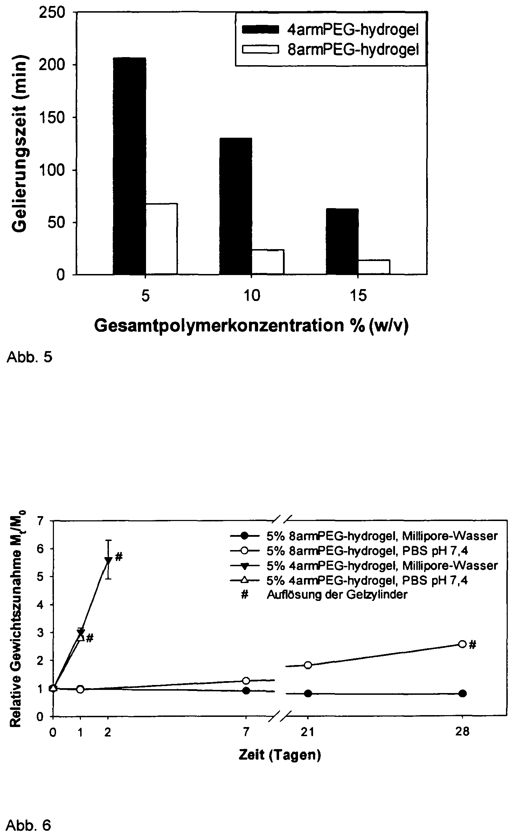

Gelierungszeit und Gelfestigkeit wurden am oszillierenden Rheometer (AR 2000 von TA Instruments, 40 mm Platte als Messgeometrie) bei 37 °C gemessen. Die Geleigenschaften wurden dabei im Wesentlichen vom Gesamtpolymergehalt und dem Verzweigungsgrad der Makromonomere bestimmt. Die Gelierungszeit nahm mit steigendem Gesamtpolymergehalt und steigendem Verzweigungsgrad ab (**Abb. 5**). So sank die Gelierungszeit von 206 min (5 % 4armPEG-Hydrogel) auf 13 min (15 % 8armPEG-Hydrogel) ab. Umgekehrt nahm die Gelfestigkeit mit steigendem Gesamtpolymergehalt und steigendem Verzweigungsgrad stark zu (**Abb. 4**). Durch Erhöhung von Gesamtpolymergehalt und Verzweigungsgrad der eingesetzten Makromonomere konnte die Gelfestigkeit von ca. 1 kPa (5 % 4armPEG-Hydrogel) auf ca. 80 kPa (15 % 8armPEG-Hydrogel) gesteigert werden.

### Quellungs- und Abbauverhalten von Hydrogelen

Zur Untersuchung des Quellungs- und Abbauverhaltens wurden Hydrogele mit dem in Beispiel 1 beschriebenen Verfahren hergestellt. Das 4armPEG-Hydrogel wurde aus mit Furyl-Resten funktionalisierten Makromonomeren aus Herstellungsbeispiel 1 und mit Maleimid-Gruppen funktionalisierten Makromonomeren aus Herstellungsbeispiel 3 hergestellt. Das 8armPEG-Hydrogel wurde aus mit Furyl-Resten funktionalisierten Makromonomeren, die entsprechend der Vorgehensweise in Herstellungsbeispiel 1, lediglich unter Verwendung von 8armPEG10k-OH, hergestellt wurden und mit Maleimid-Gruppen funktionalisierten Makromonomeren aus Herstellungsbeispiel 4 hergestellt.

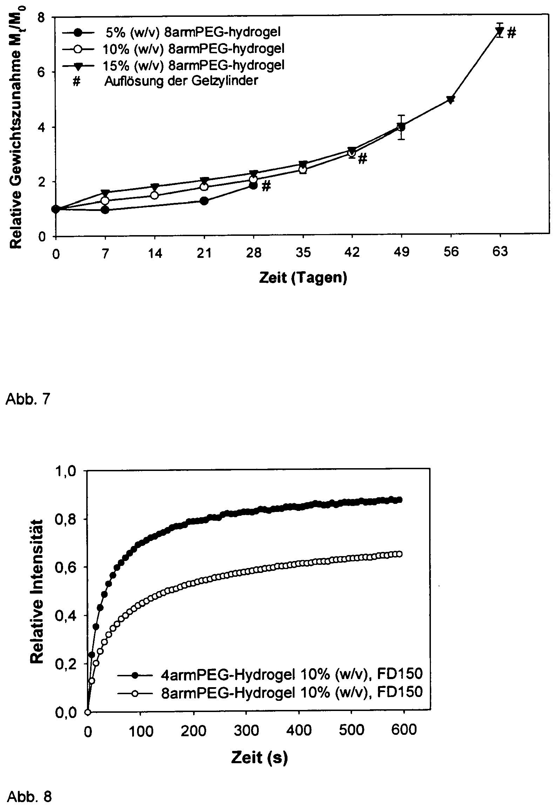

Für die Studien zum Quellverhalten wurden die flüssigen Vorläufergemische in zylinderförmige Glasformen gegossen, wo sie 72 h gelierten. Danach wurden die Proben entnommenund in unterschiedlichen Medien (Wasser und Phosphatpuffer pH 7,4) bei 37 °C inkubiert. In regelmäßigen Abständen wurde das Nassgewicht der Hydrogele bestimmt und zum ursprünglichen Gewicht in Relation gesetzt. 4armPEG-Hydrogele quellen wesentlich stärker als 8armPEG-Hydrogele; der Abbau erfolgte nach einem (Phosphatpuffer pH 7,4) bzw. zwei Tagen (Wasser). Durch Erhöhung des Verzweigungsgrad der Makromonomere konnte die Stabilität der Hydrogele deutlich erhöht werden (**Abb. 6**). 8armPEG-Hydrogele waren in Phosphatpuffer pH 7,4 über 28 Tage stabil; in Wasser konnte während des Versuchszeitraums weder Quellung noch Abbau festgestellt werden. Bei der Untersuchung des Quellungs- und Abbauverhaltens zeigte sich ebenfalls eine starke Abhängigkeit von der eingesetzten Gesamtpolymerkonzentration (**Abb. 7**). 8armPEG-Hydrogele mit 5 % (w/v), 10 % (w/v) und 15 % (w/v) Gesamtpolymergehalt waren in Phosphatpuffer pH 7,4 über 28, 42 und 63 Tage stabil.

### Diffusion von Wirkstoffen in und Freisetzung aus Hydrogelen

### Allgemeine Beschreibung

Zur Untersuchung des Diffusionsverhalten von Wirkstoffen in den erfindungsgemäßen Hydrogelen wurden FRAP-Experimente ("Fluorescence recovery after photobleaching") durchgeführt, die auch Vorhersagen über die Freisetzung der Hydrogele erlauben.

Die Methode eignet sich zur Bestimmung der Diffusionsgeschwindigkeit eines Moleküls in der Hydrogel-Matrix. Dazu wurde Fluoreszenz-gelabeltes Dextran (mittleres Molekulargewicht 150 kDa, im Folgenden auch als "FD150" bezeichnet) als Modellmolekül in das Diels-Alder-Hydrogel einpolymerisiert. Die Beobachtung der Probe erfolgte am konfokalen Mikroskop. Zunächst wurde die Fluoreszenzintensität in einem definierten Bereich bestimmt. Anschließend wurde dieser Bereich durch einen kurzen Laserimpuls gebleicht. Dabei gehen die fluoreszierenden Moleküle irreversibel in einen nichtfluoreszierenden Zustand über. Falls die Moleküle im System mobil sind, können ungebleichte, noch fluoreszierende Modellmoleküle aus der Umgebung in den gebleichten Bereich eindiffundieren. Durch Messung der Zeit bis zur Rückkehr der Fluoreszenz im gebleichten Areal kann die Diffusionskonstante bestimmt werden.

### Versuchsdurchführung

Herstellung der Testsysteme: Als Modellmolekül wurde Fluoreszenz-gelabeltes Dextran mit einem mittleren Molekulargewicht von 150 kDa (FD150) verwendet. Die eingesetzte Endkonzentration des Moleküls betrug 1 mg/mL. Als Testsysteme wurden 4arm- bzw. 8armPEG10k-Hydrogele mit einer Gesamtpolymerkonzentration von jeweils 10 % (w/v) verwendet. Die zur Herstellung der Gele benötigten Mengen der jeweiligen Gelkomponenten wurden in entsprechenden Volumina Wasser gelöst, mit FD150 versetzt, gemischt und über Nacht bei Raumtemperatur geliert.

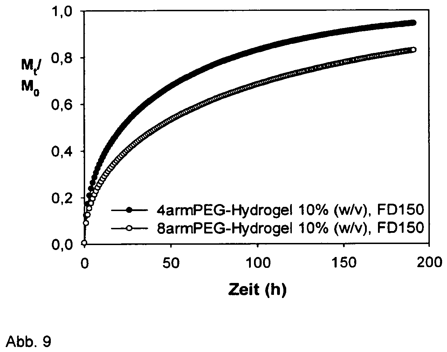

Die Durchführung der FRAP-Experimente erfolgte am konfokalen Mikroskop (Zeiss LSM 510, 10x Objektiv). Alle Experimente wurden mit der 488 nm-Linie eines 30 mW Ar-ion Lasers bei einer Ausgabeleistung von 25 % durchgeführt. Die konfokale Lochblende war dabei maximal geöffnet, um so viel Fluoreszenz wie möglich detektieren zu können. Nachdem ein Bereich im Testsystem anfokussiert wurde, wurde bei geringer Laserintensität (0,2 % Transmission) eine Zeitserie von Digitalbildern aufgenommen. Das Zeitintervall zwischen zwei aufeinanderfolgenden Bildern betrug 1,8 s. Nach Aufnahme von fünf Bildern im ungebleichten Zustand wurden jeweils kreisrunde Areale mit einem Durchmesser von 36 µm bei maximaler Laserintensität (100 % Transmission) gebleicht. Die Zeitdauer des Bleichvorgangs wurde so kurz wie möglich gewählt, um zu verhindern, dass bereits während der Bleichung eine Rückkehr der Fluoreszenz stattfindet. Im Anschluss an die Bleichung wurde eine Folge von 75 Bildern bei wiederum geringer Laserintensität aufgenommen (0,2 % Transmission), um die Rückkehr der Fluoreszenz im gebleichten Bereich in Abhängigkeit von der Zeit zu beobachten (**Abb. 8**) Anschließend wurden die aufgenommenen Bilder mit Hilfe der Software ImageJ (National Institutes of Health der Vereinigten Staaten von Amerika) ausgewertet und der zeitliche Verlauf der Fluoreszenzintensität im gebleichten Areal bestimmt. Durch Anpassung geeigneter Gleichungssysteme an die experimentellen Daten wurde der Diffusionskoeffizient D für das Modellmolekül FD150 in den verschiedenen Systemen bestimmt (vgl. Braeckmans, K. et al.; Biophys. J.,2003, 85, 2240-2252). Mit Hilfe der kalkulierten Diffusionskoeffizienten D und einem weiteren, auf dem Fickschen Gesetz basierenden Modell, wurden Vorhersagen über das Freisetzungsverhalten der Moleküle im jeweiligen System getroffen (vgl. Siepmann, J.; Siepmann, F.; Int. J. Pharm., 2008, 364, 328-343) (**Abb. 9**).

### Erläuterung der Abbildungen

Abb. 1: Schematische Darstellung der Quervernetzung von Makromonomeren mittels Diels-Alder Reaktion. Im Beispiel sind sternförmige Makromonomere mit Verzweigungsgrad vier gezeigt.
Abb. 2: Kovalent quervernetztes Hydrogel, das mit biogenen Arzneistoffen (z.B. Peptide, Proteine oder Nukleinsäuren) beladen wurde. Das Hydrogel dient als Trägersystem und ermöglicht die lokale oder systemische Therapie über einen längeren Zeitraum hinweg. Im Beispiel sind sternförmige Makromonomere mit Verzweigungsgrad vier gezeigt.
Abb. 3: Rheogramm eines Hydrogels mit 10 % (w/v) Gesamtpolymergehalt. Zur Gelherstellung wurden mit Furyl-Resten und Maleimid-Gruppen funktionalisierte PEG-Makromonomere (Molekulargewicht 10 kDa, Verzweigungsgrad vier) verwendet. Die Quervernetzung erfolgte in Wasser bei 37 °C mittels Diels-Alder Reaktion.
Abb. 4: Festigkeit der Diels-Alder-Hydrogele in Abhängigkeit von Verzweigungssgrad und Gesamtpolymerkonzentration. Je größer der Verzweigungsgrad und je höher die Gesamtpolymerkonzntration ist, desto festere Gele können gebildet werden.
Abb. 5: Gelierungszeit der Diels-Alder-Hydrogele in Abhängigkeit von Verzweigungssgrad und Gesamtpolymerkonzentration. Je größer der Verzweigungsgrad und je höher die Gesamtpolymerkonzntration ist, desto schneller setzt die Gelbildung ein.
Abb. 6: Quellungs- und Auflösungsverhalten der Diels-Alder-Hydrogelen. Die Gele zeigen unterschiedliche Stabilität in Abhängigkeit vom Verzweigungsgrad und vom verwendeten Freisetzungsmedium.
Abb. 7: Quellungs- und Auflösungsverhalten in Abhängigkeit vom eingesetzten Gesamtpolymergehalt. Durch Erhöhung des Gesamtpolymergehalts kann die Stabilität der Gelzylinder verlängert werden.
Abb. 8: Rückkehr der Fluoreszenz im gebleichten Areal über die Zeit bei einer FRAP-Analyse von erfindungsgemäßen 4armPEG-Hydrogelen und 8armPEG-Hydrogelen, die mit FD150 als Modellsubstanz beladen sind.
Abb. 9: Vorhersage über das Freisetzungsverhalten von FD150 aus 4armPEG-Hydrogelen und 8armPEG-Hydrogelen.

## Patentansprüche

1. Hydrogel, das durch kovalente Quervernetzung von Makromonomeren mittels Diels-Alder [4+2]-Cycloaddition erhältlich ist, und wobei es sich bei den Makromonomeren um Stern- oder Kammpolymere mit einem Verzweigungsgrad von zwei bis acht handelt.

2. Hydrogel nach Anspruch 1, das aus Stern- oder Kammpolymeren synthetischen Ursprungs gebildet ist.

3. Hydrogel nach Anspruch 1 oder 2, wobei es sich bei den Makromonomeren um Makromonomere mit einem Molekulargewicht von 2 - 20 kDa handelt.

4. Hydrogel nach einem der Ansprüche 1 bis 3, das unter Verwendung von Polyethylenglykol als polymeres Ausgangsmaterial für die Makromonomere erhältlich ist.

5. Hydrogel nach einem der Ansprüche 1 bis 4, wobei hydrolytisch oder enzymatisch spaltbare Sequenzen in die Hydrogelstruktur eingebaut sind.

6. Hydrogel nach einem der Ansprüche 1 bis 5, das einen Arzneistoff enthält.

7. Hydrogel nach Anspruch 6, das den Arzneistoff in kovalent gebundener Form enthält.

8. Hydrogel nach Anspruch 6 oder 7, wobei es sich bei dem Arzneistoff um ein Peptid, ein Protein oder eine Nukleinsäure handelt.

9. Hydrogel nach einem der Ansprüche 1 bis 5, das lebende Zellen enthält.

10. Hydrogel nach einem der Ansprüche 1 bis 9 geeignet zur Anwendung in einem Verfahren zur therapeutischen Behandlung des menschlichen oder tierischen Körpers, wobei die Behandlung die in situ Quervernetzung am Applikationsort im lebenden Organismus umfasst.

11. Hydrogel nach einem der Ansprüche 1 bis 9 geeignet zur Anwendung als Füllmaterial für biomedizinische Anwendungen.

12. Hydrogel nach einem der Ansprüche 6 bis 8 geeignet zur Anwendung als Trägersystem für die kontrollierte Freigabe von Arzneistoffen.

13. Hydrogel nach Anspruch 9 geeignet zur Anwendung als Gerüstmaterial für implantierte oder eingewanderte Zellen.

14. Hydrogel nach einem der Ansprüche 1 bis 9 geeignet zur Anwendung bei der Behandlung von Erkrankungen am Auge.

## Claims

1. Hydrogel, which is obtainable by covalent cross-linking of macromonomers by means of Diels-Alder [4+2] cycloaddition, and wherein the macromonomers are star or comb polymers having a degree of branching of two to eight.

2. Hydrogel according to claim 1, formed of star or comb polymers of synthetic origin.

3. Hydrogel according to claim 1 or 2, wherein the macromonomers are macromonomers having a molecular weight of 2 - 20 kDa.

4. Hydrogel according to any of claims 1 to 3, which is obtainable by using polyethylene glycol as a polymer raw material for the macromonomers.

5. Hydrogel according to any of claims 1 to 4, wherein hydrolytically or enzymatically clearable sequences are incorporated in the hydrogel structure.

6. Hydrogel according to any of claims 1 to 5, containing a drug.

7. Hydrogel according to claim 6, containing the drug in a covalently bound form.

8. Hydrogel according to claim 6 or 7, wherein the drug is a peptide, a protein or a nucleic acid.

9. Hydrogel according to any of claims 1 to 5, containing living cells.

10. Hydrogel according to any of claims 1 to 9, suitable for the use in a method for the therapeutic treatment of the human or animal body, wherein the treatment comprises the in situ cross-linkage at the application site in the living body.

11. Hydrogel according to any of claims 1 to 9, suitable for the use as filling material for biomedical applications.

12. Hydrogel according to any of claims 6 to 8, suitable for the use as carrier system for the controlled release of drugs.

13. Hydrogel according to claim 9, suitable for the use as scaffolding material for implanted or migrated cells.

14. Hydrogel according to any of claims 1 to 9, suitable for the use in the treatment of diseases of the eye.

## Revendications

1. Hydrogel pouvant être obtenu par réticulation covalente de macromonomères au moyen de la cycloaddition [4+2] de Diels-Alder, et dans lequel les macromonomères sont des polymères en étoile ou en peigne ayant un degré de ramification de deux à huit.

2. Hydrogel selon la revendication 1, constitué de polymères en étoile ou en peigne d'origine synthétique.

3. Hydrogel selon la revendication 1 ou 2, dans lequel les macromonomères sont des macromonomères ayant un poids moléculaire de 2 à 20 kDa.

4. Hydrogel selon l'une des revendications 1 à 3, pouvant être obtenu en utilisant le polyéthylène glycol comme matière de départ polymère pour les macromonomères.

5. Hydrogel selon l'une des revendications 1 à 4, dans lequel des séquences clivables hydrolytiquement ou enzymatiquement sont intégrées dans la structure de l'hydrogel.

6. Hydrogel selon l'une des revendications 1 à 5, qui contient un médicament.

7. Hydrogel selon la revendication 6, qui contient le médicament sous une forme liée de manière covalente.

8. Hydrogel selon la revendication 6 ou 7, dans lequel le médicament est un peptide, une protéine ou un acide nucléique.

9. Hydrogel selon l'une des revendications 1 à 5, qui contient des cellules vivantes.

10. Hydrogel selon l'une des revendications 1 à 9, approprié à l'utilisation dans un procédé destiné au traitement thérapeutique du corps humain ou animal, dans lequel le traitement comprend la réticulation in situ au site d'application dans l'organisme vivant.

11. Hydrogel selon l'une des revendications 1 à 9, approprié à l'utilisation comme matière de charge pour des applications biomédicales.

12. Hydrogel selon l'une des revendications 6 à 8, approprié à l'utilisation comme système support pour la libération contrôlée de médicaments.

13. Hydrogel selon la revendication 9, approprié à l'utilisation comme matière structurale pour des cellules implantées ou importées.

14. Hydrogel selon l'une des revendications 1 à 9, approprié à l'utilisation dans le traitement des maladies de l'oeil.


### SEQUENCE LISTING

<110> Universität Regensburg
<120> Herstellung von Hydrogelen mittels Diels-Alder Reaktion
<130> U2841 PCT
<150> DE 10 2011 117 839.6
   <151> 2011-11-07
<160> 3
<170> PatentIn version 3.5
<210> 1
   <211> 7
   <212> PRT
   <213> Artificial sequence
<220>
   <223> /note="Description of artificial sequence: MMP cleavable sequence"
<400> 1
   Pro Leu Gly Leu Trp Ala Arg
   1 5
<210> 2
   <211> 4
   <212> PRT
   <213> Artificial sequence
<220>
   <223> /note="Description of artificial sequence: sequence 1 for promoting cell adhesion"
<400> 2
   Arg Gly Asp Ser
   1
<210> 3
   <211> 5
   <212> PRT
   <213> Artificial sequence
<220>
   <223> /note="Description of artificial sequence: sequence 2 for promoting cell adhesion"
<400> 3
   Tyr Ile Gly Ser Arg
   1 5